# EUROPEAN PATENT SPECIFICATION

(11) **EP 0 412 513 B1**
(45) Date of publication and mention of the grant of the patent: **05.06.1996**
(21) Application number: 90115181.1
(22) Date of filing: 07.08.1990
(51) Int. Cl.: G01N 3/30

(54) **Method of inspection of products and apparatus therefor**
Verfahren zur Überwachung von Produkten und entsprechender Apparat
Méthode pour inspecter des objets et appareil correspondant

(30) Priority: 08.08.1989 JP 203973/89
(43) Date of publication of application: 13.02.1991
(73) Proprietor: AKEBONO BRAKE INDUSTRY CO., LTD., Chuo-ku Tokyo (JP); AKEBONO RESEARCH AND DEVELOPMENT CENTRE LTD., Hanyu-shi Saitama-ken (JP)
(72) Inventor: Matsuzaki, Mikio, Kasukabe-shi, Saitama (JP)
(74) Representative: Henkel, Feiler, Hänzel & Partner

(56) References cited:
- EP-A- 0 121 395
- DE-A- 3 524 943
- US-A- 4 539 561
- US-A- 4 719 583
- US-A- 4 802 366

## Description

### BACKGROUND OF THE INVENTION

### Field of the Invention

The present invention relates to a method of inspection of products and an apparatus therefor.

### Discussion of the Background

In composite material products such as plastic products, disc pads for brakes, linings and so on, due to a small difference in manufacturing conditions, cracking at a surface of product and /or cracking in the interior thereof which affect quality defects may frequently develop after forming of product. It is therefore necessary to inspect the product to see whether cracking has developed, or whether the hardness of the material exceeds a predetermined value or not.

Common conventional methods used for the inspection of products include, for example, a visual inspection of a surface of product, and striking the product by hand and listening to the tone of the sound that results. However, these inspection methods rely on human perception and are time consuming. Thus, it is impossible to inspect all of the products thoroughly. Furthermore, individual variances cannot be eliminated from human judgement, so the accuracy of the inspection is not uniform.

In inspection of hardness, such as Brinell, Vickers, and so on, inspection is performed by the use of hardness meter. These inspections can be automated if provided with predetermined long inspection times. However, efficiency is lowered, and it is also impossible to inspect all of products. Additionally, marks caused by pressing the inspection equipment are left on the surfaces of products after the inspection, and the appearance and quality of the product is deteriorated, resulting in a decline in product yields.

### SUMMARY OF THE INVENTION

It is an object of the present invention to provide an improved method and apparatus for inspecting products which overcome the aforesaid problems.

Additional objects and advantages of the invention will be set forth in part in the description which follows, and in part will be obvious from the description, or may be learned by practice of the invention. The objects and advantages of the invention may be realized and attained by means of the instrumentalities and combinations particularly pointed out in the appended claims.

To achieve the objects and in accordance with the purpose of the invention, as embodied and broadly described herein, the inspection method of this invention for inspecting the quality of a product comprises the steps of
conveying said product to a striking means at a predetermined interval by means of a conveyer ;
applying a striking force upon a surface of said product by means of a striker of said striking means ;
detecting a time period while said striker contacts said product by means of a force sensor ;
determining a characteristic of said product depending upon the time period while said striker contacts the product ;
changing the position of the striker with respect to the product selectively in a conveying direction of the conveyer or a direction perpendicular to the conveying direction of the conveyer by actuating a moving mechanism when the quality inspection of at least one portion of the product is finished; and
repeating the above steps to complete the quality inspection of a plurality of portions of the product.

Further in the achievement of the objects and in accordance with the propose of the invention, as embodied and broadly described herein, the product inspecting apparatus of the invention comprises means for conveying a product to be inspected, drive means for driving the conveying means, sensor means for detecting a position of the product on the conveying means and for controlling the drive means to stop the product at a predetermined position,
striking means comprising a striker for applying a striking force to a surface of said product stopped at said predetermined position, as known from the document EP-A-0 121 395. This apparatus is characterized by
force detecting means for detecting a time period while said striker contacts said product ; and
a moving mechanism for moving the striking means in the conveying direction of the conveyer, the direction perpendicular to the conveying direction of the conveyer or the direction perpendicular to the surface of the conveyor in order to change the position of the striking means with respect to the product.

In accordance with the invention, when the striker is pulsed downward to strike the surface of product, it is possible to determine internal characteristics of the product, on the basis of the time period while the striker contacts the product. Time period while two colliding bodies contact is shorter if they are hard, and the striking force acts only at the time of contact. When the bodies are spaced from each other, there is not force or counter force produced. That is, contact time period is inversely proportional to hardness, and the contact time period is equal to the time period the force is active. Therefore the contact time period upon striking a product can be obtained from a value of counter force detected by a force sensor, and if this contact time period is longer than a predetermined value, the existence of craking or pinhole, or the like is established. It is therefore possible to determine quality defects by this procedure.

In accordance with the product inspecting apparatus of this invention, a product is conveyed on the conveyer, and when the position detecting sensor detects arrival of the product at a predetermined position, the driving source is stopped to stop the product at the predetermined position. Next, the striking means is pulsed downward to strike the surface of the product with the striker, so that the force sensor detects the existence of the counter force produced in the striker, that is, the contact time period while the striker contacts the product. In such a way, it is possible to determine quality defects on the basis of the contact time period upon application of a strike on the product.

It is to be understood that both the foregoing general description and the following detailed description are exemplary and explanatory only and are not restrictive of the invention, as claimed.

### BRIEF DESCRIPTION OF THE DRAWINGS

The accompanying drawings which are incorporated in and constitute a part of the specification illustrate the embodiments of the invention, and, together with the description, serve to explain the objects, advantages and principles of the invention.
Fig. 1 is a perspective view illustrating an embodiment of the present invention;
Fig. 2 is a partially sectional view illustrating a striking means of the embodiment of Fig. 1;
Fig. 3 is a partially sectional view illustrating another arrangement of the striking means;
Figs 4 is a chart illustrating the relationship between contact time period of the striker and quality of the product;
Fig. 5 is a diagram illustrating the distribution of contact time period for good products A1 and A2;
Fig. 6 is a diagram illustrating the distribution of contact time period for defective products B1 to B4;
Fig. 7 is a diagram illustrating the distribution of contact time period for intermediate products C1 to C4;
Fig. 8 is a diagram illustrating the distribution of contact time period for defective product D1 having a pinhole;
Fig. 9 is a diagram illustrating the relationship between contact time period and the length of the arrows; and
Fig. 10 are graphs illustrating the relationship between the hardness of objective members and contact time period.

### DESCRIPTION OF THE PREFERRED EMBODIMENTS

Embodiments of the present invention will be described with reference to the drawings.

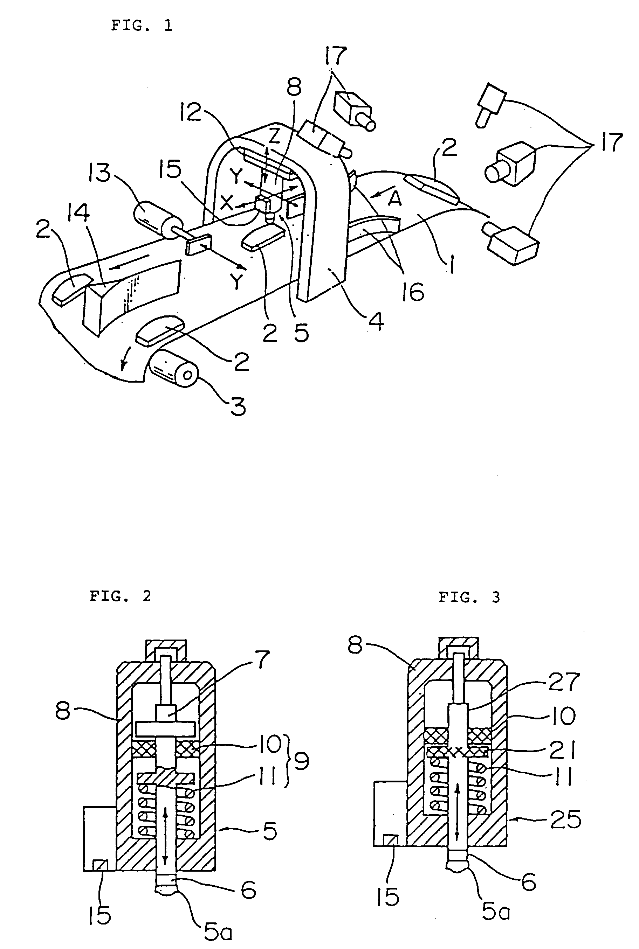

In Fig. 1, the reference numeral 1 represents a conveyer for conveying products 2, such as pads for disc brakes, at a predetermined interval in the direction of the arrow A in order that these products can be inspected. Conveyer 1 is driven and carried by rollers (not shown) which are rotated by a stepping motor 3 as a precision driving means. In an intermediate portion of conveyer 1, an arch-shaped support member 4 is provided across the width of the conveyer 1. A striking means 5 is mounted on the support member 4.

As shown in Fig. 2, the striking means 5 includes a striker 7, a contact element 5a (for example, diamond, steel, which is if necessary, hardened or coated with beryllium or the like, plastic, rubber, etc.) provided fixedly to the end portion of the striker 7, a force sensor 6 inserted between the striker 7 and the contact element 5a, a casing 8 enclosing the striker 7, and a driving mechanism 9 for giving a predetermined movement to the striker 7 which functions as an iron core held movably within and projecting from a stop end portion of the casing 8. The force sensor 6 may be a pressure sensor such as a piezo-electric type, a distortion gauge, etc. The driving mechanism 9 includes an annular electromagnet 10 and a coil spring 11. When energized, the attractive force of the electromagnet 10 is applied to the striker 7 to drive the contact element 5a downwardly a predetermined amount. When the electromagnet 10 is deenergized, the striker 7 is returned into the casing 8 by the recoil of the compressed coil spring 11. The energization of the electromagnet is brief, in the nature of a pulse. Applying such a pulsating movement to the striker 7 enables a strike force to be briefly inflicted upon the product 2 by the contact element 5a. It is possible to determine the contact time period, upon application of the strike force, between the contact element 5a and the product 2 on the basis of the output time of the force sensor 6. Alternatively, the striker 7 may be arranged to be projected by the elastic force of the coil spring 11 and returned to its initial position by the attractive force of the electromagnet 10.

The striking means 5 is mounted on the arch-shaped support member 4 through a moving mechanism 12. The position of the striking means 5, that is, the striker 7, can be changed in the respective directions X (the direction of the conveyer 1), Y (the direction perpendicular to the conveying direction of the conveyer 1) and Z (the direction perpendicular to the surface of the conveyer 1) by the moving mechanism 12. Consequently, by moving the striker 7 in the direction Z by the moving mechanism 12, it is possible to apply a strike of a predetermined force to the product 2 depending upon the thickness of the product. By moving the striker 7 in the direction X or Y, it is possible to strike each portion of the product 2, making it possible to obtain internal characteristics of the entire product 2.

A position detecting sensor 15 (e.g., laser type, supersonic wave type, etc.) is mounted on the striking means 5. Position detecting sensor 15 stops stepping motor 3 and in turn conveyer 1 when the front end of the product 2 is detected. The reference numeral 16 represents a pair of guides which guide the products 2 as they are conveyed into the striking means 5.

At the rear of the striking means 5, there are provided a plurality (five in this embodiments of VTR (videotape recorder) cameras 17 for filming the product 2 which has not yet received quality inspection by the striking means 5. Pictures taken by the cameras 17 are relayed to a cathode-ray tube (not shown) for the sake of visual pre-inspection.

In front of the striking means 5, there is provided an actuator 13 for moving the product 2 in the direction Y after the product has passed the stroking means 5. The actuator 13 is constituted by a cylinder means driven by air or oil pressure or the like. The product 2 which is regarded as defective by the VTR cameras 17 or the striking means 5, is contacted and moved by actuator 13 in the direction Y across the conveyer 1, and removed at one side of a separating board 14. The product 2 which is regarded as excellent by the striking means 5 and the cameras 17 is conveyed onward.

Fig. 3 illustrates another example of the arrangement of a striking means 25, and the same parts as those in Fig. 2 are referenced correspondingly. An annular electromagnet or permanent magnet 21 is fixed to a striker 27. Striker 27 is provided with a force sensor 6 fixed on the top end thereof adjacent the bottom of casing 8. Magnet 21 faces the electromagnet 10, with the facing sides having the same polarity. The electromagnet 10 and the magnet 21 when energized apply their repulsive forces to the striker 27, so that the striker 27 is pulsed downwardly from casing 8. When the electromagnet 10 and magnet 21 are deenergized the striker 27 is returned into the casing 8 by the elastic force of the coil spring 11, thus operating in the same manner as that of the above-mentioned striking means 5.

Although the electromagnet 10 is used as means for moving the striker 7 or 27 in the embodiments shown in Fig. 2 or 3, the present invention is not limited to this form of actuation, and for example, a cam driven, an electric motor, oil or air pressure or other means to move the striker 7 or 27 may be used. Further, if the force sensor 6 is formed by a piezo-electric element, a voltage may be applied directly to the force sensor 6.

Next the inspection operation will be described.

The product 2 is supplied onto the conveyer 1 at a predetermined interval by a supplying means (not shown) and is filmed by a plurality of the VTR cameras 17, resulting in visual pre-inspection. If a crack or other obvious defects are discovered in the product 2 during this pre-inspection, the product 2 is passed through the striking means 5 without the striking means being actuated. The actuator 13 is driven to move the product 2 in the direction Y across the conveyer 1, and passed through one side of the separating board 14 and withdrawn from the product line.

If a crack or other obvious defects are not discovered by the VTR cameras 17 during pre-inspection, the striking means 5 is actuated. That is, when the front end of the product 2 guided by guides 16 is detected by the position detecting sensor 15, the stepping motor 3 is stopped to in turn stop the conveyer 1, and product 2 is positioned just under the striker 7. Next, a current is temporarily conducted to the electromagnet 10 of the striking means 5 to activate striker 7 against the elastic force of the coil spring 11 and a strike force is applied to the product 2 through the contact element 5a. The counter force at that time is detected by the force sensor 6. This detected value is inputted and indicated on an F.F.T. (Fast Fourier Transform analyzer), an oscilloscope, etc. so as to obtain a contact time period, that is, the time to produce a counter force. On the basis of the contact time period, judgement is made as to whether the quality of the product 2 is good or not.

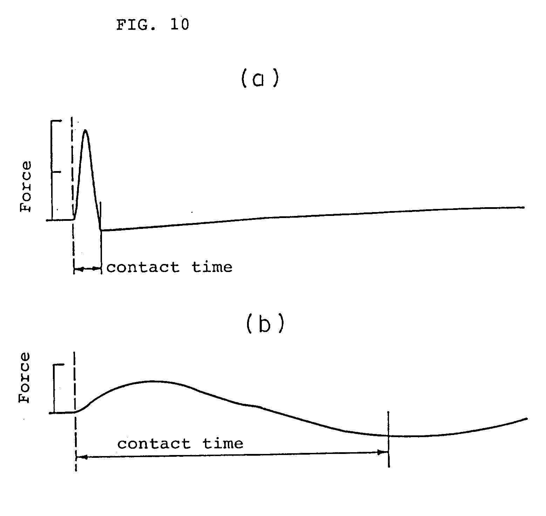

As shown in cases (a) and (b) of Fig. 10, the objective members are steel and rubber respectively. Time period while two colliding objects contact is shorter if they are hard. The striking force is active only during contact and counter force is then produced. Once the objects are separated from each other, no force or counter force is further produced. Thus, contact time period is inversely proportional to hardness, and the contact time period is set by the length of time the force is active. If the contact time period should persist longer than the predetermined contact time period as represented by Fig. 10(b), the existence of a crack or a pinhole, or the like is determined to be present.

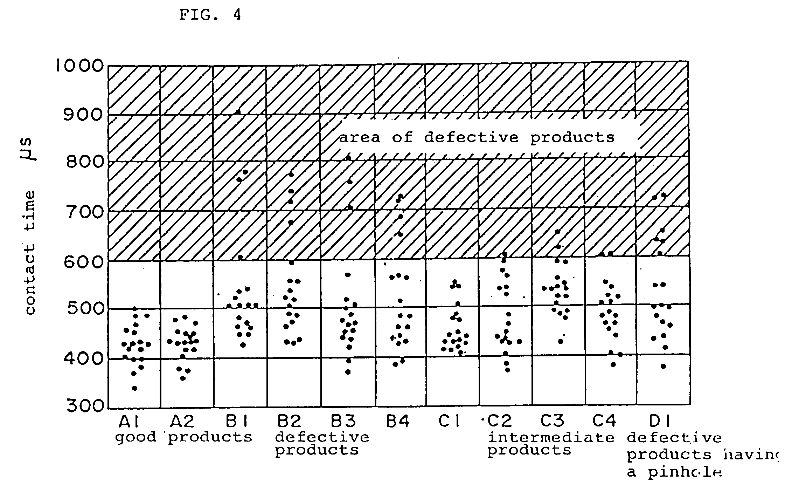

Fig. 4 illustrates whether the quality of the product 2 (disc pad) is good or not. The ordinate indicates the contact time of the contact element 5a, that is, the time of existence of a detected value by the force sensor 6, and the 20 abscissa indicates the quality of the product. As is apparent from Fig. 4, in the case where the product 2 is a typical disc pad, it was confirmed from sensory inspections and various comparison experiments performed by the present inventor that the product 2 can be judged as a good product (product A1 or A2) if the contact time is within the region from 300 to 500µs. A defective product (product B1 to B4, or D1) occurs if the contact time is obviously more than 600µs, and an intermediate product (product C1 to C3) occurs if the contact time is about 600µs. It is assumed that the boundary (about 600µs) between good and defective products changes corresponding to the type of product 2.

When the quality inspection of particular portion of the product 2 is finished and the striker 7 is returned by the elastic force of the coil spring 11, the moving mechanism 12 (Fig. 1) is actuated, so as to selectively change the position of the striker 7 in the direction X or Y, and the above-mentioned inspection upon another portion of the product 2 is performed. This operation is then repeated sufficient times to complete the inspection of the product.

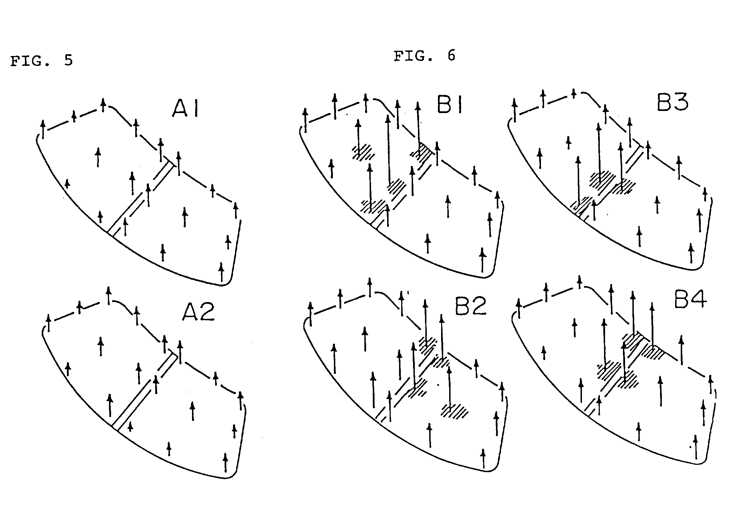

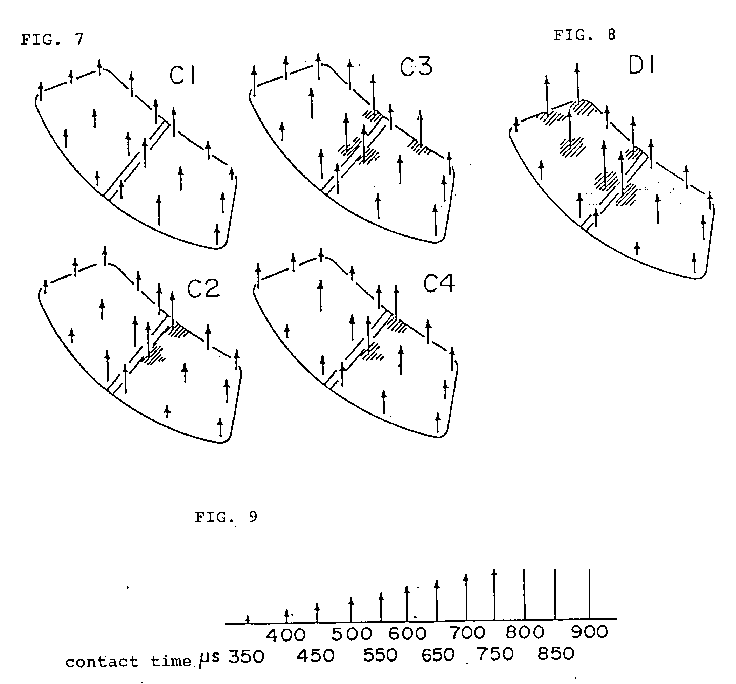

Figs. 5 to 8 show the distribution of the contact time period of the product 2 having been inspected as shown in Fig. 4. Figs. 5 to 8 respectively show good products A1 and A2, defective products B1 to B4, intermediate products C1 to C4, and a defective product D1 having a pinhole. The length of the arrows illustrated in each drawings indicates the length of the contact time from 350 to 900µs. The shadowed portions of the product 2 in the Figures are example portions in which the sensory inspection of the sound caused by striking the product is regarded as abnormal, i.e. the portion in which the contact time is more than 600µs. Striking forces were applied to eighteen portions of each product 2.

As a result of the quality inspection, if even one portion of the product is regarded as defective, the actuator 13 is actuated to press and move the product 2 in the direction Y across the conveyer 1, and product 2 is withdrawn.

In conducting inspections using the present invention, it is possible to implement in a comparatively easy manner the assembly and operation of the respective means, such as the striking means 5, the moving mechanism 12, the actuator 13, the VTR cameras 17 and so on, and to collect the data by a computer or sequential controller (not shown).

As is understood from the above description, the following effects can be obtained according to the present invention.
(1) Since there is no interference from surrounding noises, the reliability is higher than that of sensory inspection. Since the sound of striking becomes small when the node of a vibration point is hit, judgmental errors are apt to be produced as to whether the quality of the product is good or not. However, the use of a striking force and a force sensor is not influenced by the location of the node or by surrounding noise, so that the accuracy and quality of inspection is improved.
(2) On the basis of the length of the contact time accompanied with striking, it is possible to immediately judge whether there is a crack, a pinhole, and so on in a product or not, that is, whether the quality is acceptable or not. Therefore, since the determination of contact time and the measured value of force permit quality inspections in a reliable and efficient manner, automation of the quality inspection of products is attainable.
(3) By striking a plurality of portions of a product, it is possible to identify the location of the defect, and the type of defect, and then to use this information to investigate the cause of the production of defective products.
(4) Since the contact time upon application of the striking force is inversely proportional to the hardness of a product, the inspection of hardness can be performed at the same time. Since this is different from the usual sclerometer, a constant time to maintain pressure is not necessary, making it possible to shorten the time of measurement, and marks of pressure are not left in the surfaces of products, so that product yield is not decreased.
(5) It is not necessary to attach a pickup to product, so that inspection is efficiently performed.
(6) The inspection of products put on a conveyer is performed automatically. Additionally, the apparatus for the quality inspection is simple and comparatively low in price.

## Claims

1. A method for inspecting the quality of a product, comprising the steps of:
conveying said product (2) to a striking means (5, 25) at a predetermined interval by means of a conveyer (1);
applying a striking force upon a surface of said product (2) by means of a striker (7, 27) of said striking means (5, 25);
detecting a time period while said striker (7, 27) contacts said product (2) by means of a force sensor (6);
determining a characteristic of said product (2) depending upon the time period while said striker (7, 27) contacts the product (2);
changing the position of the striker (7, 27) with respect to the product (2) selectively in a conveying direction of the conveyer (1) or a direction perpendicular to the conveying direction of the conveyer (1) by actuating a moving mechanism (12) when the quality inspection of at least one portion of the product (2) is finished; and
repeating the above steps to complete the quality inspection of a plurality of portions of the product (2).

2. The method for inspecting of claim 1, further comprising the step of filming said product (2) prior to applying said striking force to visually determine if said product (2) is defective.

3. The method for inspecting of claim 2, further comprising the step of passing a defective product (2) through said striker (7, 27) without applying said striking force.

4. The method for inspecting of claim 2 or 3, further comprising the step of driving an actuator (13) to remove said defective product (2) from said conveyer (1) after said defective product (2) has passed through said striker (7, 27).

5. The method for inspecting of claim 1, further comprising the steps of sensing the position of said product (2) on said conveyer (1) and driving said striker (7, 27) when said product (2) is located thereunder.

6. A product inspecting apparatus comprising:
means (1) for conveying a product (2) to be inspected;
drive means (3) for driving said conveying means (1);
sensor means (15) for detecting a position of said product (2) on said conveying means (1) and for controlling said drive means (3) to stop said product (2) at a predetermined position; and ;
striking means (5, 25) comprising a striker (7, 27) for applying a striking force to a surface of said product (2) stopped at said predetermined position; characterized by
force detecting means (6) for detecting a time period while said striker (7, 27) contacts said product (2); and
a moving mechanism (12) for moving the striking means (5, 25) in the conveying direction of the conveyer (1), the direction perpendicular to the conveying direction of the conveyer (1) or the direction perpendicular to the surface of the conveyor (1) in order to change the position of the striking means (5, 25) with respect to the product (2).

7. The product inspecting apparatus of claim 6, further comprising filming means (17) for visually inspecting and determining if said products (2) are defective.

8. The product inspecting apparatus of claim 7, further comprising actuating means (13) for removing any defective products (2) from said conveyer (1).

9. The product inspecting apparatus of claim 6, wherein said drive means (3) is a rotary drive means.

## Patentansprüche

1. Verfahren zum Prüfen der Güte eines Erzeugnisses, umfassend die folgenden Schritte:
Transportieren des Erzeugnisses (2) zu einer Schlageinrichtung (5, 25) in einem vorbestimmten Abstand mittels eines Förderers (1),
Ausüben einer Schlagkraft auf eine (Ober-)Fläche des Erzeugnisses (2) mittels eines Schlägers oder Stößels (7, 27) der Schlageinrichtung (5, 25),
Detektieren bzw. Messen einer Zeitspanne, während welcher der Stößel (7, 27) das Erzeugnis (2) kontaktiert, mittels eines Kraftsensors (6),
Bestimmen einer Eigenschaft des Erzeugnisses (2) in Abhängigkeit von der Zeitspanne, während welcher der Stößel (7, 27) das Erzeugnis (2) kontaktiert,
selektives Ändern der Stellung des Stößels (7, 27) gegenüber dem Erzeugnis (2) in einer Transportrichtung des Förderers (1) oder einer Richtung senkrecht zur Transportrichtung des Förderers (1) durch Betätigung eines Bewegungsmechanismus (12), wenn die Güteprüfung mindestens eines Bereichs des Erzeugnisses (2) abgeschlossen ist, und
Wiederholen der obigen Schritte zur vollständigen Durchführung der Güteprüfung an mehreren Bereichen oder Teilen des Erzeugnisses (2).

2. Prüfverfahren nach Anspruch 1, ferner umfassend den Schritt eines Filmens bzw. (Bild-)Aufnehmens des Erzeugnisses (2) vor der Ausübung der Schlagkraft zwecks visueller Bestimmung, ob das Erzeugnis (2) fehlerhaft ist.

3. Prüfverfahren nach Anspruch 2, ferner umfassend den Schritt eines Vorbeiführens eines fehlerhaften Erzeugnisses (2) am (unter) dem Stößel (7, 27) ohne Ausübung der Schlagkraft.

4. Prüfverfahren nach Anspruch 2 oder 3, ferner umfassend den Schritt eines Antreibens eines Betätigungsglieds (13) zum Entfernen des fehlerhaften Erzeugnisses (2) vom Förderer (1), nachdem das fehlerhafte Erzeugnis (2) den Stößel (7, 27) passiert hat.

5. Prüfverfahren nach Anspruch 1, ferner umfassend die Schritte eines Erfassens der Lage des Erzeugnisses (2) auf dem Förderer (1) und eines Antreibens des Stößels (7, 27), wenn sich das Erzeugnis (2) darunter befindet.

6. Erzeugnisprüfvorrichtung, umfassend:
eine Einrichtung (1) zum Fördern eines zu prüfenden Erzeugnisses (2),
eine Antriebseinrichtung (3) zum Antreiben der Fördereinrichtung (1),
eine Sensoreinrichtung (15) zum Detektieren einer Stellung oder Lage des Erzeugnisses (2) auf der Fördereinrichtung (1) und zum Steuern der Antriebseinrichtung (3) für das Anhalten des Erzeugnisses (2) in einer vorbestimmten Stellung sowie
eine Schlageinrichtung (5, 25) mit einem Schläger oder Stößel (7, 27) zum Ausüben einer Schlagkraft auf eine (Ober-) Fläche des in der vorbestimmten Stellung angehaltenen Erzeugnisses (2),
gekennzeichnet durch
eine Kraftdetektiereinrichtung (6) zum Detektieren bzw. Messen einer Zeitspanne, während welcher der Stößel (7, 27) das Erzeugnis (2) kontaktiert, und
einen Bewegungsmechanismus (12) zum Bewegen der Schlageinrichtung (5, 25) in der Transportrichtung des Förderers (1), in der Richtung senkrecht zur Transportrichtung des Förderers (1) oder in der Richtung senkrecht zur Oberfläche des Förderers (1) zwecks Änderung der Stellung der Schlageinrichtung (5, 25) gegenüber dem Erzeugnis (2).

7. Erzeugnisprüfvorrichtung nach Anspruch 6, ferner umfassend Film- bzw. (Bild-)Aufnahmeeinrichtungen (17) für eine visuelle Prüfung und zum Bestimmen, ob die Erzeugnisse (2) fehlerhaft sind.

8. Erzeugnisprüfvorrichtung nach Anspruch 7, ferner umfassend ein Betätigungsmittel (13) zum Entfernen etwaiger fehlerhafter Erzeugnisse (2) vom Förderer (1).

9. Erzeugnisprüfvorrichtung nach Anspruch 6, wobei die Antriebseinrichtung (3) eine Rotationsantriebseinrichtung ist.

## Revendications

1. Procédé d'inspection de la qualité d'un produit, comprenant les étapes suivantes :
le transport du produit (2) vers un dispositif de frappe (5, 25) avec des intervalles prédéterminés, à l'aide d'un transporteur (1),
l'application d'une force de frappe à la surface du produit (2) à l'aide d'un organe de frappe (7, 27) du dispositif de frappe (5, 25),
la détection d'une période au cours de laquelle l'organe de frappe (7, 27) est au contact du produit (2) à l'aide d'un capteur (6) de force,
la détermination d'une caractéristique du produit (2) dépendant de la période pendant laquelle l'organe de frappe (7, 27) est au contact du produit (2),
le changement de la position de l'organe de frappe (7, 27) par rapport au produit (2) d'une manière sélective dans la direction de transport du transporteur (1) ou en direction perpendiculaire à la direction de transport du transporteur (1) par commande d'un mécanisme de déplacement (12) lorsque l'inspection de qualité d'une partie au moins du produit (2) est terminée, et
la répétition des étapes successives pour l'exécution de l'inspection de qualité de plusieurs parties du produit (2).

2. Procédé d'inspection selon la revendication 1, comprenant en outre l'étape de prise de l'image et du produit (2) avant l'application de la force de frappe pour la détermination visuelle du fait que le produit (2) est défectueux.

3. Procédé d'inspection selon la revendication 2, comprenant en outre l'étape de circulation d'un produit défectueux (2) au niveau de l'organe de frappe (7, 27) sans application de la force de frappe.

4. Procédé d'inspection selon la revendication 2 ou 3, comprenant en outre une étape de pilotage d'un organe de manoeuvre (13) destiné à retirer le produit défectueux (2) du transporteur (1) après que le produit défectueux (2) a circulé au niveau de l'organe de frappe (7, 27).

5. Procédé d'inspection selon la revendication 1, comprenant en outre des étapes de détection de la position du produit (2) sur le transporteur (1), et de pilotage de l'organe de frappe (7, 27) lorsque le produit (2) est placé sous celui-ci.

6. Appareil d'inspection de produits, comprenant :
un dispositif (1) de transport d'un produit (2) à inspecter,
un dispositif (3) d'entraînement du dispositif de transport (1),
un dispositif capteur (15) destiné à détecter la position du produit (2) sur le dispositif de transport (1) et à commander le dispositif d'entraînement (3) afin que le produit (2) soit arrêté en position prédéterminée, et
un dispositif de frappe (5, 25) comprenant un organe de frappe (7, 27) destiné à appliquer une force de frappe à une surface du produit (2) qui est arrêté en position prédéterminée, caractérisé par
un dispositif (6) de détection de force destiné à détecter la période pendant laquelle l'organe de frappe (7, 27) est au contact du produit (2), et
un mécanisme (12) de déplacement du dispositif de frappe (5, 25) dans la direction de transport du transporteur (1), en direction perpendiculaire à la direction de transport du transporteur (1) ou en direction perpendiculaire à la surface du transporteur (1) afin que la position du dispositif de frappe (5, 25) par rapport au produit (2) soit modifiée.

7. Appareil d'inspection de produit selon la revendication 6, comprenant en outre un dispositif (17) de prise d'image destiné à l'inspection visuelle et à la détermination du fait que les produits (2) sont défectueux.

8. Appareil d'inspection de produit selon la revendication 7, comprenant en outre un dispositif de commande (13) destiné à retirer les produits défectueux éventuels (2) du transporteur (1).

9. Appareil d'inspection de produit selon la revendication 6, dans lequel le dispositif d'entraînement (3) est un dispositif d'entraînement en rotation.
